Europäisches Patentamt

(19) European Patent Office

Office européen des brevets

(11) Numéro de publication : **0 279 713 B1**

# (12) FASCICULE DE BREVET EUROPEEN

(45) Date de publication du fascicule du brevet :
**12.06.91 Bulletin 91/24**

(51) Int. Cl.⁵ : **B01J 8/04, C01C 1/04, C07C 29/15**

(21) Numéro de dépôt : **88400064.7**

(22) Date de dépôt : **13.01.88**

(54) **Procédé et appareil pour effectuer sous pression des réactions chimiques dans une zône réactionnelle multi-étagée avec conditionnements thermiques intermédiaires extérieurs.**

(30) Priorité : **21.01.87 FR 8700751**

(43) Date de publication de la demande :
**24.08.88 Bulletin 88/34**

(45) Mention de la délivrance du brevet :
**12.06.91 Bulletin 91/24**

(84) Etats contractants désignés :
**BE DE ES GB IT NL**

(56) Documents cités :
**DE-A- 2 929 300
FR-A- 2 573 996
GB-A- 1 140 071
GB-A- 2 120 119**

(73) Titulaire : **INSTITUT FRANCAIS DU PETROLE
4, Avenue de Bois-Préau
F-92502 Rueil-Malmaison (FR)**

(72) Inventeur : **Vu, Quang Dang
48 bis, Boulevard du Général Leclerc
F-92200 Neuilly (FR)**
Inventeur : **Ham, Pierre
Domaine Saint François d'Assise Les
Choucas No. 2
F-78170 La Celle Saint Cloud (FR)**
Inventeur : **Gelas, Daniel
3, avenue du Colifichet
F-78290 Croissy Sur Seine (FR)**
Inventeur : **Legrand, Christian
45, rue Serpente
F-95800 Cergy (FR)**

Jouve, 18, rue Saint-Denis, 75001 PARIS

## Description

L'invention concerne un procédé pour effectuer sous pression des réactions chimiques à partir de fluides réactionnels en présence d'au moins un catalyseur solide, dans une zone réactionnelle multi-étagée avec conditionnements thermiques intermédiaires extérieurs. Elle concerne aussi le réacteur pour la mise en oeuvre du procédé et l'utilisation du réacteur et du procédé.

L'invention est applicable notamment du méthanol, de l'ammoniac ainsi qu'au réformage catalytique, à l'hydrogénation stabilisatrice des essences et à l'hydrocraquage des coupes pétrolières lourdes.

Plus généralement, elle est applicable dans tous les procédés de la catalyse hétérogène où des réactifs fluides, soit liquides, soit gazeux, soit les uns liquides et les autres gazeux sont amenés à réagir entre eux, dans une succession de plusieurs lits catalytiques constitués par des grains, granulés, sphères ou autres éléments solides à structures plus ou moins complexes et élaborées.

Les réactions concernées se produisent généralement avec des chaleurs réactionnelles nécessitant un ou plusieurs réajustements thermiques intermédiaires avec le milieu extérieur.

Lorsqu'on veut réaliser des réactions chimiques à forte chaleur de réaction, il est connu de le faire en plusieurs étapes avec un rajustement thermique intermédiaire après chacune d'elles.

Ainsi, dans le réformage catalytique des essences par exemple (brevet FR-A-2 160 269 – US-A-4 210 519 – US-A-4 233 268), on dispose généralement de trois réacteurs à lits de catalyseur solide séparés par deux fours de réchauffage extérieurs.

Cette multiplicité des réacteurs est coûteuse en équipement, tuyauterie et espace d'implantation.

C'est la raison pour laquelle il a été souvent proposé de regrouper les différents réacteurs dans une seule et unique enceinte réactionnelle dont les parois sont calculées pour pouvoir bien résister à la pression interne relativement élevée du système.

Dans cette solution, les lits de catalyseur solide sont superposés verticalement et reposent soit sur des grilles de soutien soit directement sur des cloisons de séparation horizontales. (FR-A-2.573.996).

Ces grilles et cloisons doivent supporter, d'une part le poids du lit catalytique, d'autre part des contraintes nées de la perte de charge due à la circulation du fluide réactionnel à travers les équipements de transfert thermique et le lit catalytique lui-même.

Il résulte de l'addition de ces deux contraintes, des charges extrêmement élevées, de l'ordre de 50 à 100 et même 150 tonnes au mètre carré alors que les spécifications courantes pour planchers industriels se limitent autour de 0.5 à 1 tonne au mètre carré.

Pour éviter ce cumul des efforts qui amène à des solutions mécaniques contraignantes tant du point de vue du poids des poutrelles de soutien que de celui de l'espace mort perdu, il a été proposé, notamment pour des réacteurs de méthanol (Hydrocarbon Processing, Mai 1984 p 95-100) une superposition, non plus de réacteurs axiaux, mais de réacteurs radiaux où, le catalyseur étant placé dans une cartouche cylindrique creuse, voire dans plusieurs cartouches, le fluide réactionnel circule horizontalement soit de façon centrifuge du cylindre intérieur au cylindre extérieur soit de façon centripète du cylindre extérieur vers le cylindre intérieur.

Cette solution qui a l'avantage de décharger les planchers de séparation, des charges de contraintes dues à la circulation du fluide réactionnel, a par contre, l'inconvénient de laisser beaucoup d'espace mort notamment un noyau central vide que le brevet GB-A-1140071 enseigne de remplir avec un échangeur de chaleur.

Un inconvénient de cette solution souligné par le brevet US-A-4225562 résulte de la difficulté de bien centrer les deux cylindres délimitant la couronne de catalyseur solide. De ce décentrage, résulte une hétérogénéité dans les parcours des filets fluides, extrêmement préjudiciable à la bonne réalisation de la réaction de base et qui conduit à des dépassements thermiques mettant en danger la stabilité du catalyseur.

Un autre inconvénient tient à l'obligation de chargement et de déchargement des cartouches catalytiques. A cet effet, on peut installer des brides de même diamètre que celui du réacteur mais cette solution n'est pas la meilleure lorsqu'on opère sous pression et que la taille du réacteur est importante. Il est alors recommandé de souder les extrémités généralement hémisphériques au corps cylindrique mais pour effectuer le déchargement du catalyseur, il est nécessaire de scier chaque fois les dites extrémités pour retirer les cartouches et de les resouder après la phase de chargement du réacteur. L'opération est longue, délicate, soumise à chaque fois à une autorisation et un contrôle administratifs.

Par ailleurs, le brevet US-A-4225562 enseigne de prévoir des compartiments parallélipipédiques disposés parallèlement à l'axe de l'enceinte et ayant tous la même section et le même volume.

Le dispositif utilise au mieux la moitié de l'espace disponible. Outre la complexité mécanique et donc le coût important du dispositif, la standardisation des compartiments de volume identique interdit l'adaptation du volume catalytique à la variation de la vitesse de réaction. De ce fait, le brevet ne peut s'appliquer qu'aux réacteurs soit à un seul étage cinétiquement parlant soit réalisant des réactions dont la vitesse ne varie pas en fonction de la conversion c'est-à-dire à des réactions d'ordre zéro.

En ce qui concerne les différentes variantes de réacteurs à cloisons radiales on peut signaler le dispositif enseigné par le brevet US 3898049, où la cartouche catalytique est divisée en plusieurs secteurs dans le sens longitudinal. Ces secteurs sont parcourus successivement par les fluides réactionnels, alternativement de haut en bas et de bas en haut suivant l'axe longitudinal de l'enceinte réactionnelle. Il est aisé de se rendre compte que le dispositif proposé n'est applicable que dans des cas très particuliers où la longueur du parcours et, par voie de conséquence, les pertes de charge ont peu d'importance car, par rapport à un réacteur axial de même hauteur, et à fortiori par rapport à un réacteur radial, le trajet du fluide est considérablement rallongé et proportionnel au nombre de sections que comporte la cartouche.

Par ailleurs l'art antérieur est illustré par le brevet GB 2.120.119 qui décrit un réacteur longitudinal pour la réalisation de synthèses chimiques en phase gazeuse. Ce réacteur comprend des enceintes parallélépipédiques contenant du catalyseur disposées selon l'axe du réacteur et ayant des parois opposées perméables à travers lesquelles circulent la charge. L'effluent peut être refroidi intérieurement par un quench, ce qui limite l'espace disponible interne, d'autant plus que l'homogénéisation du mélange obtenu implique un grand volume de l'injecteur à l'entrée de chaque compartiment. Par ailleurs, ce quench qui dilue la concentration des réactifs a pour effet de ralentir la cinétique de réaction et d'augmenter la demande en volume catalytique du système. Cet effet est d'autant plus accentué lorsque le quench est constitué par une partie de la charge fraîche.

Le brevet FR-A-2.573.996 illustre aussi un réacteur catalytique à compartiment unique pour la synthèse d'ammoniac et de méthanol dépourvu d'échangeurs internes et dont la température des parois est maintenue à un niveau peu élevé par la présence d'un matelas d'air.

Enfin, le brevet DE-A-2.929.300 décrit un réacteur échangeur à écoulement axial dans des zones catalytiques à section variable, ces zones étant non adajacentes.

Un des premiers objets de l'invention est donc de proposer un procédé et un réacteur multi-étagé, sous pression avec conditionnements thermiques intermédiaires extérieurs, qui ne présentent pas les inconvénients mentionnés ci-dessus. Notamment un objet de l'invention est de proposer un réacteur, peu coûteux qui soit de construction facile et d'une manipulation aisée pour le chargement et le déchargement du catalyseur.

Un autre objet est la réalisation d'un réacteur où sensiblement tout le volume disponible peut être occupé par le catalyseur.

Encore un autre objet de l'invention est de pouvoir assurer un meilleur contrôle de la répartition des vitesses du fluide à travers les lits catalytiques de façon à éviter les surchauffes locales, ce qui ne manquerait pas de désactiver le catalyseur et donc de diminuer sa durée d'utilisation et de perturber le degré d'avancement de la réaction.

D'autres objets de l'invention et des avantages nouveaux apparaitront lors de la description ci-après. Plus particulièrement, l'invention concerne un procédé pour effectuer sous pression des réactions chimiques dans une zone réactionnelle cylindrique à base de section circulaire comprenant au moins deux compartiments renfermant chacun au moins en partie au moins un catalyseur solide, dans lequel on introduit un fluide réactionnel dans au moins un compartiment, on fait circuler ledit fluide réactionnel dans ledit compartiment, on récupère un effluent réactionnel dudit compartiment, on échange de la chaleur entre l'effluent devant entrer dans au moins un compartiment subséquent et un milieu d'échange de chaleur, on introduit ensuite ledit effluent ayant échangé de la chaleur dans ledit compartiment subséquent, on fait circuler ledit effluent dans le compartiment et l'on récupère un autre effluent réactionnel dudit compartiment subséquent, ladite zone réactionnelle étant de forme allongée et comportant une enveloppe avec au moins une génératrice, ladite enveloppe définissant une section fermée se situant dans un plan coupant ladite génératrice,

le procédé étant caractérisé en ce qu'on fait entrer le fluide réactionnel et l'effluent réactionnel dans lesdits compartiments par au moins une zone de distribution, on fait circuler ledit fluide réactionnel et ledit effluent réactionnel dans les compartiments transversalement de façon sensiblement perpendiculaire à ladite génératrice, lesdits compartiments étant de forme allongée suivant ladite génératrice et étanches, chacun desdits compartiments étant adjacent à un ou deux autres compartiments, chaque groupe de deux compartiments adjacents comportant une paroi commune ou mitoyenne soit sensiblement parallèle à ladite génératrice, soit sensiblement oblique par rapport à ladite génératrice, on récupère l'effluent réactionnel dans au moins une zone de collecte,

le procédé étant en outre caractérisé en ce qu'on échange de la chaleur à l'extérieur de la zone réactionnelle entre l'effluent et un milieu d'échange de chaleur externe.

Dans ces conditions le réacteur présente un bon compromis remplissage et niveau de perte de charges et de plus permet que le fluide ait des trajets réguliers dans le lit catalytique.

Au lieu d'une paroi mitoyenne continuellement oblique, le réacteur peut comporter une paroi comprenant des sections décalées les unes par rapport aux autres dans la même direction, chacune d'elles étant parallèle à la génératrice, mais dont l'ensemble constitue l'équivalent d'une paroi oblique.

Toutes ces sections de paroi sont reliées entre elles de façon à être étanches aux fluides. Cette caractéristique présente l'avantage de faire varier l'épaisseur du lit catalytique dans le réacteur pour améliorer la distribution du fluide dans la masse catalytique sur toute sa hauteur.

La zone réactionnelle est avantageusement cylindrique à base circulaire.

La longueur de chaque compartiment et de chaque paroi mitoyenne est sensiblement celle de la zone réactionnelle et la section en coupe des compartiments peut s'inscrire sensiblement dans la section de l'enveloppe.

Lorsque la zone réactionnelle (section de l'enveloppe) est avantageusement circulaire, la section des compartiments peut s'inscrire dans le cercle défini par la section de l'enveloppe ou dans un cercle qui lui est coaxial.

Tous les compartiments sont hydrodynamiquement isolés et étanches de sorte qu'il n'y a aucune communication possible entre les divers compartiments à l'intérieur de la zone réactionnelle par quelque paroi que ce soit. Le fluide ou l'effluent réactionnel pénètre habituellement dans un compartiment par une entrée connectée à une zone de distribution intérieure au compartiment et en sort après avoir traversé transversalement tout le lit catalytique, par une issue connectée à une zone de collecte intérieure généralement située à l'opposé de la zone de distribution.

Le nombre de compartiments et par conséquent d'échanges de chaleur intermédiaires est variable suivant les cas d'espèces. La section et/ou le volume des compartiments peut être identique ou différent et est avantageusement déterminé en fonction des paramètres et des contraintes du procédé à réaliser, par exemple la vitesse de réaction qui est fonction du degré d'avancement de la conversion.

L'invention concerne aussi le dispositif pour la mise en oeuvre du procédé.

Plus généralement, il comprend :
– un réacteur (2) susceptible de contenir au moins un catalyseur, présentant une enveloppe (10) substantiellement cylindrique définie par au moins une génératrice et comportant une première extrémité (30) et une seconde extrémité étanches (31),
– au moins deux compartiments (201, 203) adjacents, contenus dans ledit réacteur de forme allongée suivante ladite génératrice, chaque groupe de deux compartiments adjacents comportant une paroi (210) mitoyenne ou commune, chaque compartiment comportant en outre au moins un moyen d'entrée (1) du fluide réactionnel ou de l'effluent réactionnel dans ledit compartiment et au moins un moyen de sortie (3) de l'effluent réactionnel qui a traversé ledit compartiment, lesdits moyens d'entrée et de sortie étant en communication avec l'extérieur du réacteur à travers ladite enveloppe (10), caractérisé en ce que lesdits compartiments sont étanches, ladite paroi mitoyenne ou commune étant soit sensiblement parallèle à la génératrice soit sensiblement oblique à celle-ci et en ce que le réacteur comporte :
– au moins un moyen de distribution (20) dans chaque compartiment adapté à distribuer transversalement dans chaque compartiment le fluide réactionnel ou l'effluent réactionnel de façon sensiblement perpendiculaire à ladite génératrice et relié audit moyen d'entrée,
– au moins un moyen de collecte (21) de l'effluent réactionnel de chaque compartiment relié audit moyen de sortie dudit compartiment, et,
– au moins un moyen externe de transfert de chaleur (4) interposé entre ledit moyen de sortie (3) de l'effluent réactionnel d'un des compartiments et le moyen d'entrée (8) de l'effluent réactionnel dans le compartiment suivant (203).

Un autre moyen de transfert ou d'échange de chaleur consiste selon un autre mode de réalisation, à procéder par mélange à l'aide de gaz et avantageusement de fluide ou d'effluent réactionnel frais et relativement froid, à un refroidissement de l'effluent à sa sortie d'au moins un compartiment et avant son envoi dans un compartiment suivant ce qui présente l'avantage de supprimer un ou plusieurs échangeurs. A cette fin, le réacteur présente généralement des moyens d'introduction de ce gaz pour réaliser cette trempe (ou "quench") ces moyens pouvant être à l'intérieur ou à l'extérieur du réacteur et de préférence à l'extérieur.

La zone réactionnelle ou enceinte du réacteur, de forme allongée, de préférence sensiblement cylindrique, est généralement divisée, dans le sens de la longueur, avantageusement suivant une direction sensiblement parallèle à la génératrice, en plusieurs compartiments, par des cloisons étanches, qui peuvent être reliées directement à l'enveloppe de l'enceinte. La longueur des cloisons étanches suivant la génératrice est généralement égale à la longueur du réacteur.

Les compartiments ainsi définis, préalablement remplis de catalyseur solide, sont parcourus successivement par le fluide réactionnel qui est soit un gaz, soit un liquide soit un mélange gaz-liquide.

Le fluide et de manière plus précise l'effluent réactionnel, après avoir partiellement réagi sur le lit catalytique d'un des compartiments, sort de l'enceinte, traverse un appareil de transfert thermique qui le reconditionne à la température adéquate et repénètre dans l'enceinte pour passer sur l'étage catalytique suivant. Le volume de chaque compartiment est généralement ajusté de façon que dans chaque compartiment la variation de température entre l'entrée et la sortie de compartiment soit comprise entre environ 1 et 200°C et de préférence entre 5 et 150°C.

Ce parcours du fluide engendre des pertes de charge et crée des différences de pression entre les compartiments mitoyens de l'enceinte. Ces écarts de pression dynamiques exercent sur les parois des compartiments, des contraintes mécaniques qui peuvent être considérables et qui peuvent résulter en des épaisseurs de métal difficilement acceptables.

Les parois mitoyennes des différents compartiments peuvent être soit des panneaux plans supportés, verticaux ou obliques, soit avantageusement des panneaux de forme courbe, auto-portante. Ils peuvent être avantageusement, sensiblement parallèles à la génératrice de la zone réactionnelle.

Lorsque les panneaux sont plans et supportés, de préférence les supports sont des tirants placés sensiblement perpendiculairement aux plans des panneaux et reliés d'une part à la cloison, d'autre part à la paroi de l'enceinte réactionnelle.

Lorsque les panneaux sont de forme autoportante, de préférence, ils comportent soit des assemblages de tôles profilées, soit de secteurs cylindriques dont les génératrices sont sensiblement parallèles à la génératrice du réacteur et dont les base sont soit des arcs de cercle dont le rayon est compris entre 0,1 et 100 fois, de préférence entre 0,5 et 50 fois, celui de l'enceinte réactionnelle, soit des portions de courbes quadratiques telles que parabole, hyperbole ou ellipse.

A l'intérieur des compartiments remplis d'éléments de catalyseur solide, le fluide réactionnel ou l'effluent réactionnel est introduit et réparti à travers la masse catalytique, au moyen de distribution constituent la zone de distribution.

Les filets fluides ainsi formés, après avoir traversé le lit catalytique, sont rassemblés et amenés vers la sortie grâce à des collecteurs généralement situés à l'opposé des distributeurs, qui constituent la zone de collecte.

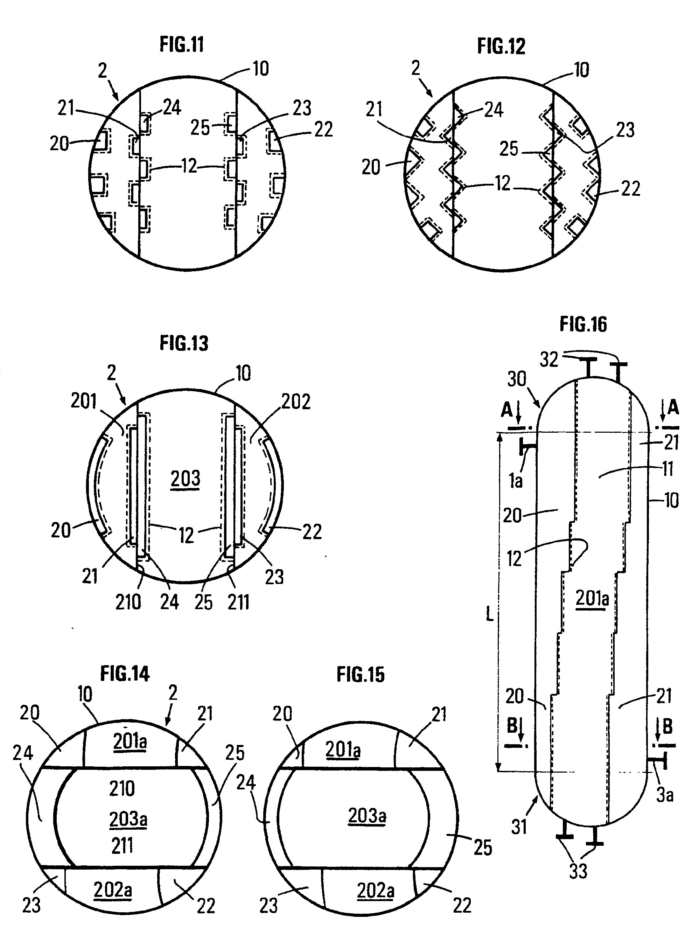

Les distributeurs et les collecteurs sont disposés de telle manière que le fluide réactionnel (et l'effluent) circule non pas dans le sens longitudinal de la zone réactionnelle, ce qui rallonge et augmente considérablement le parcours des fluides ainsi que les pertes de charge qui en résultent mais transversalement de façon perpendiculaire à l'axe du cylindre formant l'enceinte. Le parcours total du fluide à travers les lits catalytiques peut généralement rester inférieur à deux fois la longueur L, de tangente à tangente de l'enveloppe ou de la paroi cylindrique de la zone réactionnelle, de préférence inférieur ou égal à une fois cette même longueur L. (Voir fig 16 définie ci-dessous).

A cet effet, les distributeurs et collecteurs peuvent être constitués par des tuyaux dont les sections sont soit des cercles, des secteurs de cercle, de polygone ou de plan soit encore des sections communément désignés sous le nom de "scallops". L'aire des sections peut varier le long de la génératrice en fonction du débit de fluide passant à son niveau.

Les tuyaux peuvent être soit parallèles à la génératrice du réacteur soit obliques par rapport à cette génératrice.

Les moyens d'entrée et de sortie du fluide ou de l'effluent réactionnels peuvent être disposés n'importe où sur chaque compartiment. Ils peuvent cependant être avantageusement situés de telle façon que les moyens d'entrée soient situés sensiblement au niveau d'une des deux extrémités du réacteur (ou du compartiment) et que les moyens de sortie soient situés sensiblement au niveau de l'autre extrémité. Selon ce mode de réalisation, la section des tuyaux de distribution à l'entrée dans le compartiment est avantageusement supérieure à celle du même tuyau de distribution à l'autre extrémité du compartiment en raison des débits plus importants de gaz au niveau de l'entrée et inversement la section du tuyau de collecte est avantageusement plus petite au niveau de cette entrée en raison des plus faibles débits de gaz collectés à ce même niveau, que celle du même tuyau de collecte à l'extrémité de sortie du compartiment.

Cette disposition permet de maintenir une vitesse sensiblement constante de gaz en tout point du distributeur et du collecteur, ce qui permet aussi d'optimiser le remplissage en catalyseur du compartiment.

Pour atteindre cet objectif, selon un mode de réalisation particulier, la paroi du distributeur et du collecteur peut comprendre sur les parties faisant face au lit catalytique soit au moins une section de distribution et de collecte (une grille par exemple) sensiblement parallèle à la génératrice, soit au moins une section de distribution et de collecte sensiblement oblique par rapport à ladite génératrice, chacune de ces sections de distribution et de collecte étant décalée dans la même direction, l'une par rapport à l'autre de façon que les fluides traversent une épaisseur de lit catalytique constante.

Le nombre de distributeurs peut être supérieur ou inférieur au nombre de collecteurs. De préférence, le nombre de distributeurs est le même que le nombre de collecteurs, ce qui permet une bonne répartition du débit gazeux.

Lorsque les distributeurs et collecteurs d'un compartiment sont en nombre réduit, égal ou inférieur à trois, ils peuvent être disposés contre la paroi de l'enceinte près des jonctions entre paroi de l'enceinte et parois mitoyennes. Cette solution est particulièrement intéressante pour traiter des débits faibles et est très simple de mise en oeuvre. La circulation du fluide et de l'effluent réactionnel est alors sensiblement parallèle aux parois mitoyennes.

De préférence, lorsque les distributeurs ou collecteurs sont en nombre égal ou supérieur à quatre, ils sont répartis le long des parois mitoyennes et le cas échéant sur les parties de paroi de l'enceinte faisant face à ces mêmes parois mitoyennes. Cet agencement permet de traiter des débits importants de fluide et d'effluent réactionnels. Il impose une circulation du fluide et de l'effluent réactionnel de manière sensiblement perpendiculaire aux parois étanches mitoyennes des compartiments.

Pour répartir ou collecter le fluide réactionnel, les distributeurs et collecteurs peuvent être, soit percés de trous, soit constitués par des grilles comprenant des ouvertures formées à partir de fils métalliques, soit croisés, soit parallèles et dont les profils peuvent être étudiés pour faciliter au maximum l'écoulement du fluide.

Dans certain compartiments, le parcours moyen d'un filet fluide d'un distributeur à un collecteur, ou distance entre distributeur et collecteur peut être identique d'un distributeur à un autre, mais ce parcours moyen d'un filet fluide peut être variable d'un distributeur à un autre.

De préférence, la section d'ouverture sur le lit catalytique du distributeur et du collecteur lui faisant face est variable en fonction de la longueur du parcours moyen reliant ces éléments c'est-à-dire à la distance distributeur-collecteur lui faisant face.

Ces différentes caractéristiques ainsi que d'autres éléments faisant intégralement partie de l'invention sont décrits plus amplement dans la description qui suit.

Suivant un mode de réalisation, le fluide réactionnel et les effluents peuvent circuler de façon sensiblement parallèle à la paroi mitoyenne qui délimite deux compartiments adjacents, ce qui est avantageux lorsque les volumes de catalyseur dans les différents compartiments sont sensiblement du même ordre.

Suivant un autre mode de réalisation du procédé, la circulation peut s'effectuer de façon sensiblement perpendiculaire à ladite paroi notamment quand les distributeurs ou collecteurs sont situés sur cette paroi.

Ce mode de réalisation est particulièrement avantageux lorsque les volumes de catalyseur dans les différents compartiments sont très différents.

La vitesse des fluides dans les compartiments est généralement comprise entre 1 et 200 m/s et de préférence comprise entre 5 m/s et 100 m/s et dépend bien entendu de la réaction et des conditions opératoires choisies.

La températures des fluides dans les divers compartiments est généralement comprise entre 100 et 800°C, de préférence entre 200 et 600°C.

L'invention sera mieux comprise et les avantages apparaitront nettement au vu des exemples et des différentes figures illustrant schématiquement l'appareil et le procédé à titre non limitatif, parmi lesquelles :

– Les figures 1a, 1b, 1c et 1d représentent des modes de réalisation différents du procédé et du réacteur selon l'invention,

– Les figures 2, 3, 4, 5 et 6 montrent différents types de parois mitoyennes étanches,

– Les figures 7, 8, 9, 10, 11, 12 et 13 illustrent différents types de distributeurs et collecteurs et différents modes de circulation du fluide et de l'effluent réactionnel,

– Les figures 14 et 15 représentent une coupe transversale partielle sensiblement au niveau de chaque extrémité du réacteur suivant AA et BB, et

– La figure 16 représente une coupe longitudinale du réacteur.

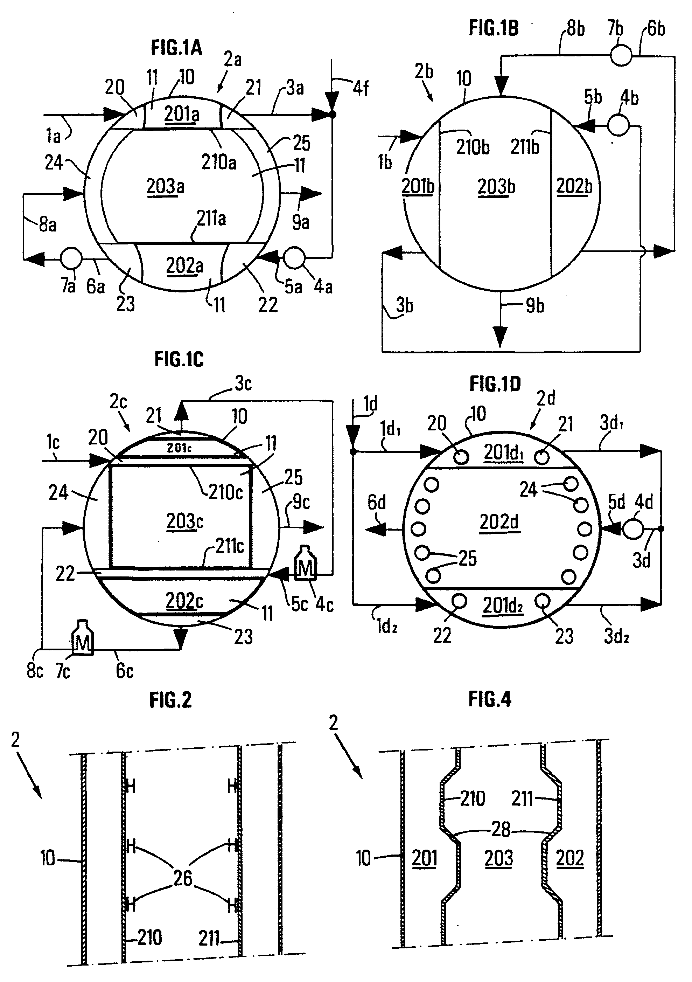

Dans les figures 1a, 1b, 1c, et 1d, on a représenté les mêmes éléments du dispositif selon l'invention par les mêmes références suivies des lettres a, b, c et d.

A titre d'exemple, dans l'application de l'appareil de l'invention à la synthèse du méthanol (figure 1a) un mélange comprenant principalement l'hydrogène et des oxydes de carbone est amené par le conduit d'entrée 1a sur le haut du réacteur 2a contenant du catalyseur du type de ceux décrits dans le livre "Applied Industrial Catalysis" (Volume 2, Chap 6, p 226 et suivantes).

Le réacteur 2a qui est un cylindre vertical comprenant une enveloppe 10 de section cylindrique et de base circulaire fermé aux deux bouts par des fonds ellipsoides ou de préférence par des fonds hémisphériques (30 et 31 fig 16) est partagé en trois compartiments sans communication interne, 201a, 202a et 203a, de volumes respectivement croissants VI < V2 < V3 au moyen de deux parois internes planes, étanches (210a et 211a) qui peuvent être fixées par exemple par soudage à l'enveloppe 10 et aux fonds 30 et 31 disposés, sensiblement parallèlement à la génératrice du réacteur 2a.

Le catalyseur a été chargé par le fond supérieur du réacteur dans les divers compartiments. L'ensemble des sections en coupe des compartiments s'inscrit dans l'enveloppe 10 de base circulaire.

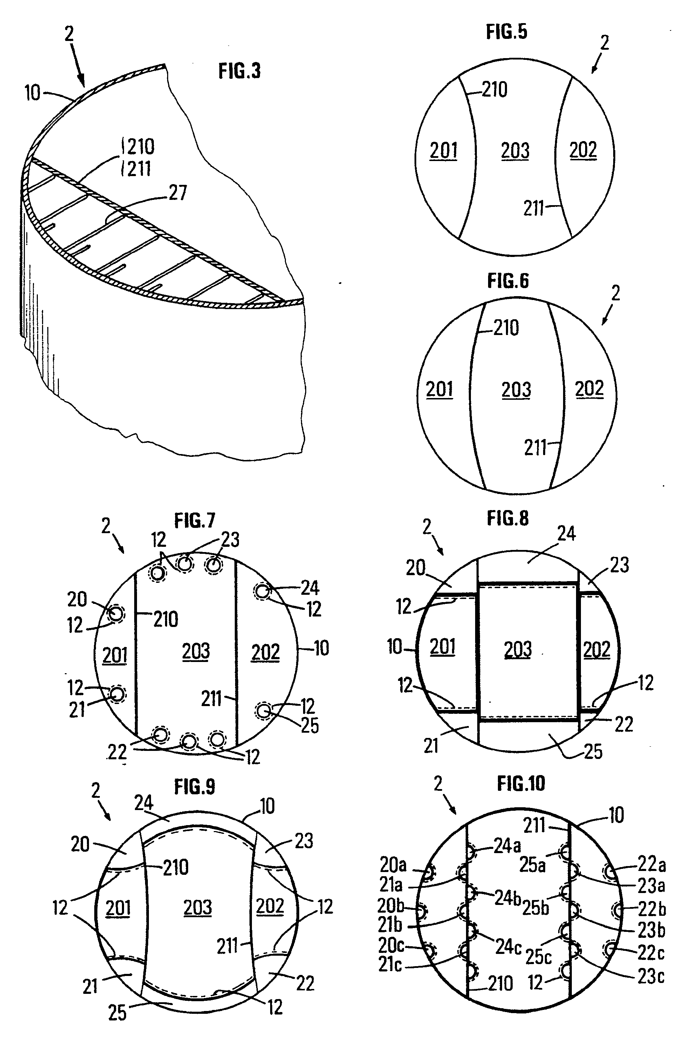

Le fluide réactionnel, préalablement conditionné du point de vue de la pression à 8 MPa et du point de vue de la température aux alentours de 250°C est introduit dans le compartiment 201a de façon sensiblement parallèle à la paroi mitoyenne par au moins un distributeur 20 percé d'ouvertures 12 (fig 8) généralement dispersées autour de sa paroi externe faisant face au collecteur. Au contact du catalyseur 11 dans le compartiment, l'hydrogène se combine avec les oxydes de carbone pour donner le méthanol selon les équations :

$$CO + 2H_2 \rightarrow CH_3OH \quad (1)$$

$$CO_2 + 3H_2 \rightarrow CH_3OH + H_2O \quad (2)$$

Les réactions (1) et (2) sont exothermiques et au fer et à mesure que la conversion progresse, la température du fluide ou de l'effluent réactionnel augmente.

Lorsque l'élévation de la température de l'effluent réactionnel atteint 20 à 60°C ce qui correspond à une présence d'environ 1 à 3% volume de méthanol dans le gaz, celui-ci est prélevé de 201a par au moins un collecteur 21 percé d'ouvertures (fig 8) ou à grille 12 et amené par le conduit 3a situé dans la partie basse vers un moyen de transfert de chaleur 4a connue en soi, par exemple un réfrigérant extérieur, qui le ramène sensiblement à sa température initiale au moment de l'entrée dans 201a. Au lieu d'un échange thermique sur 4a, on peut effectuer une trempe grâce à des moyens d'introduction 4f d'au moins une partie du fluide réactionnel ou de l'effluent froid sur le conduit amenant l'effluent vers le compartiment suivant.

Du réfrigérant 4a qui peut être, soit un aéroréfrigérant, soit un récupérateur calorifique préchauffant par exemple tout en partie de la charge ou produisant de l'eau chaude ou de la vapeur d'eau sous pression, le mélange gazeux est ramené par le conduit 5a sur le haut du réacteur 2a où il est introduit par au moins un distributeur 22 dans le compartiment 202a symétriquement opposé au compartiment 201a.

Lorsque, du fait de la réaction, la température du gaz est à nouveau relevée de 20 à 60°C par rapport à sa température d'entrée, l'effluent réactionnel est à nouveau soutiré par au moins un collecteur 23 dans la partie basse du réacteur, amené par le conduit 6a, sur le réfrigérant 7a qui le reconditionne, à nouveau, à la température optimale initiale. De 7a, le fluide emprunte le conduit 8a à la partie supérieure du réacteur pour parvenir au compartiment 203a du réacteur 2a par au moins un distributeur 24.

A la sortie de 203a, le mélange qui contient finalement entre 2 à 12% en volumes de méthanol est livré par le collecteur 25 et par le conduit 9a dans la partie basse du réacteur à l'unité de condensation et de purification de l'alcool synthétisé.

Suivant cette figure 1a, on fait circuler le fluide et l'effluent réactionnel transversalement de façon sensiblement perpendiculaire à la génératrice du réacteur et de façon parallèle à la paroi mitoyenne.

Les compartiments 201a, 202a, 203a peuvent être disposés côte à côte, avec le compartiment 202a occupant la position centrale. Cet ordre minimise les efforts de contrainte exercés par les différences de pression sur les parois mitoyennes 210a, 211a.

Une disposition préférée est que le dernier compartiment 203a (fig 1a) occupe une position centrale par rapport aux deux premiers 201a et 202a.

Des études comparatives ont montré en effet que c'est cette disposition qui est la plus favorable au point de vue de l'optimisation de l'espace utile disponible.

Dans la description ci-dessus (fig 1a), le réacteur 2a est disposé de façon verticale.

Lorsque les conditions locales l'imposent, par exemple, lorsque le sol est spongieux et peu résistant, le réacteur 2 peut être disposé horizontalement suivant un autre mode de réalisation comme l'indique la figure 1b.

Dans cette alternative, de préférence, les conduits 1b, 3b, 5b, 6b, 8b et 9b sont placés de telle sorte que la circulation du fluide réactionnel se fait de manière sensiblement parallèle à la paroi mitoyenne et de haut en bas, ce qui évite les dangers de déstabilisation des lits catalytiques par soufflage des grains de catalyseur.

Sur la figure 1c illustrant un autre mode de réalisation, on a représenté un système réactionnel destiné au réformage catalytique des essences, qui est endothermique. Le schéma illustre en même temps un exemple où le fluide et l'effluent réactionnels circulent perpendiculairement aux parois 210c, 211c, dans les compartiments 201 c et 202 c, et où ils circulent parallèlement à ces mêmes parois dans le compartiment 203c.

Dans le circuit 1c, circulent des vapeurs de naphta ainsi que de l'hydrogène recyclé. Le mélange de gaz et de vapeur de naphta est préalablement conditionné sous environ 0,5 à 3 MPa à environ 500 à 530°C. Dans le compartiment 201 c du réacteur 2c, le catalyseur de réformage qui peut être du platine déposé sur support acide et dopé par divers autres métaux nobles, comme par exemple le rhénium, fait subir au naphta essentiellement des réactions de déshydrogénation qui abaissent fortement la température du fluide réactionnel, jusqu'aux alentours de 450-480°C. Ce fluide est alors extrait de 201c pour venir par le conduit 3c sur le four extérieur 4c, connu en soi, qui le ramène à nouveaux aux alentours de 500°C.

Le fluide est ensuite envoyé par le conduit 5c sur le compartiment 202c. Dans le compartiment 202c, aux réactions de déhydrogénation, viennent s'ajouter des réactions de réarrangement de la molécule et l'abaissement de la température est beaucoup moins important.

Lorsque cet abaissement atteint autour de 10 à 30°C, le fluide est sorti du deuxième compartiment 202 cet réchauffé à nouveau aux alentours de 500°C dans le four 7c.

Le troisième compartiment 203c est pratiquement isotherme. En effet, aux réactions de déshydrogénation et de réarrangement moléculaires sont venues s'ajouter des réactions d'hydrogénation qui sont exothermiques et dont la chaleur de réaction compense pratiquement celle du réformage proprement dit.

Les procédés de l'invention selon les figures 1a, 1b et 1c s'appliquent aux réacteurs dont les différents compartiments peuvent être connectés en série de façon à constituer une succession d'étages au sens cinétique du mot.

Ils s'appliquent également aux réacteurs multiétagés dont certains compartiments peuvent être connectés en parallèle (figure 1d) pour former ensemble soit le premier soit le dernier étage réactionnel.

Ce mode de réalisation particulier peut être avantageux dans certains cas, notamment en synthèse de l'ammoniac.

En effet, on sait que l'on peut opérer actuellement cette synthèse sous des conditions relativement douces (pression autour de 10 MPa et température autour de 400°C) et que sous ces conditions, il n'est plus nécessaire de prévoir une double enveloppe pour refroidir la paroi extérieure de l'enceinte réactionnelle.

Par contre, il se pose toujours pour ces réacteurs à paroi non refroidie dits "réacteurs à paroi chaude" le problème des pertes de charge dues au grand débit volumique des gaz à travers le lit catalytique.

Le fait de pouvoir diviser le débit du gaz par deux aux endroits critiques permet de contourner cette difficulté.

Sur la figure 1d, on a représenté le schéma d'un réacteur pour synthèse d'ammoniac, réalisé selon le procédé de l'invention.

Un mélange de gaz de synthèse contenant environ 25% d'azote, 75% d'hydrogène, des traces d'ammoniac et d'argon (< 5%) sont introduits sous environ 10 MPa et à 350-400°C dans le conduit 1d. Le conduit 1d est prolongé par deux conduits 1d1 et 1d2 qui amènent ce gaz dans les deux compartiments 201d1 et 201d2, avantageusement de volumes égaux et de sections égales, et disposés symétriquement par rapport à l'axe du cylindre formant volume réactionnel.

Grâce à cette mise en parallèle, le volume du premier étage catalytique, bien que faible par rapport au débit volumique du gaz de synthèse, n'offre finalement que peu de résistance au passage gazeux.

A la sortie des compartiments en parallèle 201 d1 et 201 d2, le gaz de synthèse contient environ 4 à 7% d'ammoniac et sa température s'est élevée d'environ 40 à 60°C.

Par les conduits 3d1 et 3d2 les débits de gaz aboutissent au conduit 3d qui débouche sur l'échangeur récupérateur de chaleur 4d.

A la sortie de l'échangeur 4d, les gaz refroidis à la température optimale d'environ 350-400°C sont amenés par le conduit 5 d sur le compartiment central 202 d du réacteur 2d.

Les gaz effluents du réacteur 2d, qui contiennent environ 8 à 14% en volumes d'ammoniac et qui se trouvent approximativement à 400-450° et sous environ 10 MPa, sont envoyés par le conduit 6d à l'unité de conditionnement et de récupération d'ammoniac située en aval de la section réactionnelle.

Dans les réacteurs ainsi décrits, (fig 1a, 1b, 1c) les cloisons de séparation 210-211, si elles sont déchargées du poids du catalyseur qui repose directement sur la paroi de l'enceinte, ont cependant à résister en général à la poussée due à la différence de pression entre compartiments mitoyens 201-203 d'une part, 203-202 d'autre part.

Ces différences de pression qui sont dues à la perte de charge à travers les lits catalytiques et les appareils de transfert thermiques sont de l'ordre de 0,1 à 0,4 MPa et peuvent même atteindre 1MPa dans le cas où interviennent des fours de réchauffage comme avec le réformage par exemple.

Dans l'exemple du réacteur pour synthèse du méthanol représenté par la figure 1a, la paroi 210a qui a par exemple 3200 mm de large et 20 mm d'épaisseur est prévue pour résister à une différence de pression de 0,3 MPa.

Lorsqu'on emploie un système de supportage parallèle au plan de cette paroi on peut utiliser des poutrelles 26 du type HEM 400 réparties par exemple tous les 500 mm (figure 2).

Avec un système de supportage perpendiculaire, on peut utiliser par exemple, des tirants 27 de 25 mm de diamètre, disposés selon un maillage carré de 500 mm de côté, les tirants étant reliés d'une part à la cloison 210, d'autre part à la paroi de l'enceinte réactionnelle 10 (figure 3).

La paroi plane avec ses systèmes de supportage relativement encombrants et difficiles à installer peut être remplacée par une paroi simple mais ayant une forme spécialement étudiée 28 lui permettant de pouvoir résister seule contre la pression. Lorsqu'on adopte une forme autoportante, on peut employer notamment des profilés de même épaisseur par exemple que celle de la paroi plane 210 (20 mm), du type indiqué sur la figure 4 et dont la hauteur par exemple peut être de 300 mm et le pas de 395 mm.

Les figures 5 et 6 représentent d'autres formes autoportantes de l'invention avec convexité et concavité orientées vers les compartiments 201 et 202. La combinaison de ces diverses formes peut aussi se rencontrer. Ces formes sont préférées à celles présentées sur la figure 4 car elles facilitent la liaison entre ces parois et la paroi cylindrique du réacteur.

Les parois 210, 211 sont des secteurs de cylindre dont les génératrices sont sensiblement parallèles à la génératrice de l'enceinte réactionnelle et dont les bases sont des arcs de cercle dont le rayon est compris entre 0,1 et 100 fois, de préférence entre 0,5 et 50 fois, celui de l'enceinte réactionnelle.

Dans les réacteurs selon l'invention, les fluides réactionnels circulent transversalement de façon perpendiculaire à la génératrice de l'enceinte. A cet effet, les distributeurs 20, 22, 24 et collecteurs 21, 23, 25 sont constitués de préférence par des tuyaux sensiblement parallèles à cette génératrice, qui présentent des ouvertures 12 généralement dispersées autour de leurs parois en direction du lit catalytique.

De préférence, la section de tuyaux de distribution 20, 22, 24 et/ou collecte 21, 23, 25 peut adopter une des formes représentées sur les figures, soit une section circulaire (fig 7), soit un secteur formé à l'endroit de la jonction entre la paroi réactionnelle et la paroi de séparation mitoyenne et fermé par un plan (fig 8) ou par un morceau de cylindre à base circulaire (Fig 9) ; la circulation des fluides s'effectue alors de façon sensiblement parallèle aux parois mitoyennes.

Lorsque la section totale des distributeurs ou celle des collecteurs représente plus que 10%, de préférence, plus que 20% de celle réservée au catalyseur, les distributeurs 20, 22, 24 et collecteurs 21, 23, 25 sont de préférence réalisés en plusieurs tuyaux (fig 10, 11 et 12) sensiblement parallèles et répartis le long de la paroi de séparation 210 mitoyenne et le cas échéant le long de la partie de paroi de l'enceinte réactionnelle lui faisant 211 ; la circulation s'effectue alors sensiblement perpendiculairement aux parois mitoyennes.

Ces différents tuyaux de distribution 20a, 20b, 20c... du premier compartiment traversé, 22a, 22b, 22c,... du deuxième compartiment traversé et 24a, 24b, 24c du troisième compartiment traversé ainsi que les différents tuyaux de collecte 21a, 21b, 21c..., 23a, 23b, 23c..., 25a, 25b, 25c respectivement du premier, deuxième et troisième compartiment peuvent avoir la forme de secteurs de cercle (Fig 10) ou de polygones par exemple de triangle (fig 12), de carré ou rectangle (fig 11). Cette même figure 11 illustre le cas où le nombre de distributeurs 24 est inférieur au nombre de collecteurs 25... On aurait pu tout aussi bien réaliser un réacteur où le nombre de collecteurs est inférieur au nombre de distributeurs.

La figure 13 représente un autre mode de réalisation des distributeurs et collecteurs soit en arc de cercle 20, 22 contre l'enveloppe, soit plans 21, 23, 24, 25 contre les parois mitoyennes 210 et 211 ;

Selon ces divers modes de réalisation, une plus grande section des ouvertures 12 compense le nombre plus réduit de distributeurs ou de collecteurs.

Les ouvertures 12 pour l'introduction et la collecte des fluides peuvent avoir une forme quelconque, circulaire par exemple, et elles ont avantageusement leur aire proportionnelle au parcours moyen correspondant à chaque couple distributeur-collecteur de façon à minimiser les passages préférentiels des fluides gazeux.

Comme le montrent les figures 14, 15 et 16, illustrant un mode de réalisation des moyens de distribution et de collecte, le conduit 1a de la figure 1a est sensiblement situé au niveau de l'une des extrémités 30 du réacteur ou du premier compartiment 201a tandis que le conduit 3a de sortie est sensiblement situé au niveau de l'autre extrémité 31 du réacteur ou du premier compartiment. La section du tuyau de distribution par exemple 20, dans le compartiment 201a au niveau de l'extrémité 30 (fig 14 coupe AA) est supérieure à la section de distribution 20 au niveau de l'extrémité 31 (fig 15 coupe BB) tandis que la section du tuyau de collecte 21 au niveau de l'extrémité 30 est inférieure à celle du tuyau de collecte 21 au niveau de l'extrémité 31. Il en est de même pour les sections des autres tuyaux de distribution 22, 24 et de collecte 23, 25 dans les autres compartiments (fig 14, 15). Selon la figure 16 illustrant de manière plus particulière ce mode de réalisation, les parois du distributeurs 20 et du collecteur 21 faisant face au lit catalytique peuvent comprendre des sections (grilles à trous 12 par exemple) sensiblement parallèles à la génératrice qui sont décalées les uns par rapport aux autres dans la même direction de sorte que l'épaisseur du lit traversé par les fluides est la même tout le long du réacteur et que les dites parois sont globalement obliques par rapport à la génératrice.

Les opérations de chargement du catalyseur peuvent s'effectuer généralement par au moins une ouverture 32 sur le fond supérieur du réacteur et de préférence par au moins une ouverture par compartiment. Dans le cas de réacteurs horizontaux à parois mitoyennes verticales 210b et 211b (figure 1b), le chargement peut s'effectuer par des ouvertures généralement disposées sur l'enveloppe du réacteur au droit de chaque compartiment.

Le déchargement s'effectue généralement par le bas au moins une ouverture d'évacuation 33 et de préférence par au moins une ouverture dans chaque compartiment. Ces opérations de chargement et de déchargement sont faciles à réaliser et n'immobilisent pas longtemps le réacteur.

Les parois du réacteur (celles de l'enveloppe et/ou les parois mitoyennes) peuvent être isolées thermiquement. On peut introduire un fluide intermédiaire, de chauffage ou de refroidissement qui peut éventuellement circuler dans les cloisons notamment dans les parois mitoyennes de compartiments adjacents.

Dans les figures ci-dessus, on a seulement représenté un réacteur à trois compartiments et deux moyens de transfert thermique pour une raison de clarté. Il est évident que l'on peut construire selon l'invention un réacteur avec une pluralité de compartiments par exemple de 3 à 10 et de moyens de transfert thermique intermédiaires.

## Revendications

1. Procédé pour effectuer sous pression des réactions chimiques dans une zone réactionnelle cylindrique à base de section circulaire comprenant au moins deux compartiments renfermant chacun au moins en partie au moins un catalyseur solide, dans lequel on introduit un fluide réactionnel dans au moins un compartiment, on fait circuler ledit fluide réactionnel dans ledit compartiment, on récupère un effluent réactionnel dudit compartiment, on échange de la chaleur entre l'effluent devant entrer dans au moins un compartiment subséquent et un milieu d'échange de chaleur, on introduit ensuite ledit effluent ayant échangé de la chaleur dans ledit compartiment subséquent, on fait circuler ledit effluent dans le compartiment et l'on récupère un autre effluent réactionnel dudit compartiment subséquent, ladite zone réactionnelle étant de forme allongée et comportant une enveloppe avec au moins une génératrice, ladite enveloppe définissant une section fermée se situant dans un plan coupant ladite génératrice, le procédé étant caractérisé en ce qu'on fait entrer le fluide réactionnel et l'effluent réactionnel dans lesdits compartiments par au moins une zone de distribution, on fait circuler ledit fluide réactionnel et ledit effluent réactionnel dans les compartiments transversalement de façon sensiblement perpendiculaire à ladite génératrice, lesdits compartiments étant de forme allongée suivant ladite génératrice et étanches, chacun desdits compartiments étant adjacent à un ou deux autres compartiments, chaque groupe de deux compartiments adjacents comportant une paroi commune ou mitoyenne soit sensiblement parallèle à ladite génératrice, soit sensiblement oblique par rapport à ladite génératrice, on récupère l'effluent réactionnel dans au moins une zone de collecte, le procédé étant en outre caractérisé en ce qu'on échange de la chaleur à l'extérieur de la zone réactionnelle entre l'effluent et un milieu d'échange de chaleur externe.

2. Procédé selon la revendication 1 dans lequel on introduit le fluide réactionnel sensiblement au niveau d'une première extrémité de la zone réactionnelle, on fait circuler le fluide réactionnel et l'effluent réactionnel dans la zone de distribution de section progressivement décroissante, on fait circuler l'effluent réactionnel qui a traversé le compartiment dans la zone de collecte de section progressivement croissante et on récupère l'effluent réactionnel au niveau de la seconde extrémité de la zone réactionnelle.

3. Procédé selon l'une des revendications 1 et 2 dans lequel on échange de la chaleur en procédant à un mélange desdits effluents réactionnels avec du gaz relativement froid.

4. Procédé selon l'une quelconque des revendications 1 à 3 dans lequel on fait circuler ledit fluide et ledit effluent dans lesdits compartiments de façon que le parcours total dans la zone réactionnelle soit inférieur à deux fois la longueur L de ladite zone réactionnelle.

5. Procédé selon l'un quelconque des revendications 1 à 4 dans lequel on fait circuler le fluide réactionnel et l'effluent réactionnel de façon sensiblement parallèle à la dite paroi mitoyenne.

6. Procédé selon l'un quelconque des revendication 1 à 5 dans lequel on fait circuler le fluide réactionnel et l'effluent réactionnel de façon sensiblement perpendiculaire à ladite paroi mitoyenne.

7. Procédé selon l'une quelconque des revendications 1 à 6 dans lequel la zone réactionnelle est horizontale.

8. Procédé selon l'une quelconque des revendications 1 à 7 dans lequel la zone réactionnelle est verticale.

9. Procédé selon la revendication 7 dans lequel on fait circuler le fluide réactionnel et l'effluent réactionnel de haut en bas dans lesdits compartiments.

10. Procédé selon l'une quelconque des revendications 1 à 9 dans lequel la zone réactionnelle comprend au moins trois compartiments et dans lequel on fait circuler le fluide réactionnel en parallèle dans deux compartiments.

11. Procédé selon l'une quelconque des revendications 1 à 9 dans lequel on fait circuler le fluide réactionnel puis l'effluent réactionnel dans une succession de compartiments de volume de plus en plus grand.

12. Procédé selon l'une quelconque des revendications 1 à 9 et 11 dans lequel on fait circuler le fluide réactionnel et/ou l'effluent réactionnel dans chacun des compartiments reliés en série.

13. Procédé selon l'une quelconque des revendications 1 à 12 dans lequel la vitesse des gaz est comprise entre 1m/s et 200/ms.

14. Appareil pour effectuer sous pression des réactions chimiques comprenant :

    – un réacteur (2) susceptible de contenir au moins un catalyseur, présentant une enveloppe (10) substantiellement cylindrique définie par au moins une génératrice et comportant une première extrémité (30) et une seconde extrémité étanches (31),

    – au moins deux compartiments (201, 203) adjacents, contenus dans ledit réacteur de forme allongée suivante ladite génératrice, chaque groupe de deux compartiments adjacents comportant une paroi (210) mitoyenne ou commune, chaque compartiment comportant en outre au moins un moyen d'entrée (1) du fluide réactionnel ou de l'effluent réactionnel dans ledit compartiment et au moins un moyen de sortie (3) de l'effluent réactionnel qui a traversé ledit compartiment, lesdits moyens d'entrée et de sortie étant en communication avec l'extérieur du réacteur à travers ladite enveloppe (10),

caractérisé en ce que lesdits compartiments sont étanches, ladite paroi mitoyenne ou commune étant soit sensiblement parallèle à la génératrice soit sensiblement oblique à celle-ci et en ce que le réacteur comporte :

    – au moins un moyen de distribution (20) dans chaque compartiment adapté à distribuer transversalement dans chaque compartiment le fluide réactionnel ou l'effluent réactionnel de façon sensiblement perpendiculaire à ladite génératrice et relié audit moyen d'entrée,

    – au moins un moyen de collecte (21) de l'effluent réactionnel de chaque compartiment relié audit moyen de sortie dudit compartiment, et,

    – au moins un moyen externe de transfert de chaleur (4) interposé entre ledit moyen de sortie (3) de l'effluent réactionnel d'un des compartiments et le moyen d'entrée (8) de l'effluent réactionnel dans le compartiment suivant (203).

15. Appareil selon la revendication 14 dans lequel lesdits compartiments présentent des sections en coupes qui s'inscrivent dans l'enveloppe substantiellement cylindrique du réacteur.

16. Appareil selon l'une des revendications 14 et 15 dans lequel l'enveloppe (10) du réacteur a une base de section circulaire.

17. Appareil selon l'une des revendications 14 à 16 dans lequel ladite paroi mitoyenne (210) comprend des panneaux plans supportés.

18. Appareil selon l'une des revendications 14 à 16 dans lequel ladite paroi mitoyenne (210) comprend des panneaux de forme autoportante.

19. Appareil selon la revendication 18 dans lequel les panneaux de forme courbe autoportante présentent une base soit en arc de cercle dont le rayon est compris entre 0,1 et 100 fois celui du réacteur soit en portion de courbes quadratiques.

20. Appareil selon l'une quelconque des revendications 14 à 19 dans lequel ledit moyen de distribution et ledit moyen de collecte sont disposés de façon sensiblement parallèle à la dite génératrice, dans chaque compartiment.

21. Appareil selon l'une des revendications 14 à 20 dans lequel lesdits moyens d'entrée (1) et de sortie (3) sont respectivement situés sensiblement au niveau de la première extrémité (30) et de la seconde extrémité (31) du réacteur, caractérisé en ce que la section du moyen de distribution (20 fig 14) au niveau de ladite première extrémité est supérieure à la section du moyen de distribution, (20 fig 15) au niveau de la seconde extrémité et en ce que la section du moyen de collecte (21 fig 14) au niveau de la première extrémité est inférieure à celle du moyen de collecte (21 fig 15) au niveau de la seconde extrémité.

22. Appareil selon l'une des revendications 14 à 21 dans lequel lesdits moyens de distribution et de collecte comprennent soit au moins une section de distribution et de collecte sensiblement parallèle à ladite génératrice soit au moins une section de distribution ou de collecte sensiblement oblique par rapport à ladite génératrice, chacune de ces sections étant décalée l'une par rapport à l'autre dans la même direction tout le long du réacteur.

23. Appareil selon l'une des revendications 14 à 22 dans lequel ledit moyen de distribution (20) et ledit moyen de collecte (21) comportent une pluralité d'ouvertures (12) disposées le long desdits moyens et dont les sections respectives sont sensiblement proportionnelles à la distance entre ledit moyen de distribution et ledit moyen de collecte.

24. Appareil selon l'une des revendications 14 à 23 dans lequel le réacteur est horizontal et comporte au moins une paroi mitoyenne (210 b, fig 1b) sensiblement verticale.

25. Appareil selon l'une des revendications 14 à 24 comportant des moyens d'introduction (4f) de gaz froids disposés à la sortie de l'effluent réactionnel d'un compartiment avant son entrée dans le compartiment subséquent.

26. Utilisation du procédé selon l'une des revendications 1 à 13 ou de l'appareil selon l'une des revendications 14 à 25 pour la synthèse de l'ammoniac à partir d'hydrogène et d'azote ou pour la synthèse du méthanol ou d'alcools homologues supérieurs à partir d'hydrogène et d'oxydes de carbone ou pour le réformage catalytique des essences.

## Claims

1. A process for conducting chemical reactions in a cylindrical reaction zone essentially of circular cross-section, comprising at least two compartments, each of which contains, at least partly, at least one solid catalyst, wherein a reaction fluid is introduced into at least one compartment and is circulated therethrough, a reaction effluent is recovered from said compartment, heat is exchanged between the effluent having to enter into at least one subsequent compartment and a heat exchange medium, said first effluent, after heat exchange, is introduced into said subsequent compartment and is circulated therethrough and a second reaction effluent is recovered from said subsequent compartment, said reaction zone being of elongate shape and comprising a casing with at least one generatrix, said casing defining an enclosed section located in a plane intersecting said generatrix, said process being characterized in that the reaction fluid and the reaction effluent are entered into said compartments through at least one distribution zone, said reaction fluid and said reaction effluent are circulated through the compartments, cross-wise and substantially perpendicularly to said generatrix, said compartments being of elongate shape along said generatrix and tight, each of said compartments being adjacent to one or two other compartments, each group of two adjacent compartments comprising a common partition-wall either substantially parallel to said generatrix or substantially oblique with respect thereto and the reaction effluent is recovered through at least one collecting zone, the process being further characterized in that heat is exchanged outside the reaction zone between the effluent and an external heat exchanged medium.

2. A process according to claim 1, wherein the reaction fluid is introduced substantially at the level of a first end part of the reaction zone, the reaction fluid and the reaction effluent are circulated through the distribution zone with a progressively decreasing section, the reaction effluent, after having passed through the compartment is circulated through the collecting zone with a progressively increasing section and the reaction effluent is recovered at the level of a second end part of the reaction zone

3. A process according to one of claims 1 and 2, wherein heat exchange is performed by forming a mixture of said reaction effluents with relatively cold gas.

4. A process according to any one of claims 1 to 3, wherein said fluid and said effluent circulate through said compartments so that the total travel through the reaction zone be less than twice the length L of said reaction zone.

5. A process according to any one of claims 1 to 4, wherein the reaction fluid and the reaction effluent circulate substantially parallelly to said common partition-wall.

6. A process according to any one of claims 1 to 5, wherein the reaction fluid and the reaction effluent circulate substantially perpendicularly to said common partition-wall.

7. A process according to any one of claims 1 to 6, wherein the reaction zone is horizontal.

8. A process according to any one of claims 1 to 6, wherein the reaction zone is vertical.

9. A process according to claim 7, wherein the reaction fluid and the reaction effluent circulate downwardly through said compartments.

10. A process according to any one of claims 1 to 9, wherein said reaction zone comprises at least three compartments and wherein the reaction fluid circulates in parallel through two compartments.

11. A process according to any one of claims 1 to 9, wherein the reaction fluid and then the reaction effluent circulate through successive compartments of increasing volume.

12. A process according to any one of claims 1 to 9 and 11, wherein the reaction fluid and/or the reaction effluent circulate through serially connected compartments.

13. A process according to any one of claims 1 to 12, wherein the gas velocity ranges from 1m/s to 200 m/s.

14. An apparatus for carrying out the process according to any one of claims 1 to 13, comprising :
  – a reactor (2) adapted to contain at least one catalyst, comprising a substantially cylindrical casing (10) defined by at least one generatrix and having a first (30) and a second (31) tight end parts,
  – at least two tight adjacent compartments (201, 203) contained in said reactor of elongate shape, along said generatrix, each group of two adjacent compartments comprising a common partition-wall (210), each compartment further comprising at least one inlet means (1) for the reaction fluid or the reaction effluent in said compartment and at least one outlet means (3) for the reaction effluent having passed through said compartment, said inlet and outlet means communicating with the outside of the reactor through said casing (10),

characterized in that said compartments are tight, said common partition-wall being either substantially parallel to the generatrix or substantially oblique with respect thereto and in that the reactor comprises :
  – at least one distribution means (20) in each compartment adapted to cross-wise distribute in each compartment the reaction fluid or reaction effluent substantially perpendicularly to said generatrix and connected to said inlet means,
  – at least one collecting means (21) for the reaction effluent of each compartment, connected to said outlet means of said compartment, connected to said outlet means of said compartment, and
  – at least one external heat transfer means (4) interposed between said outlet means (3) for the reaction effluent of one of the compartments and the inlet means (8) of the reaction effluent in the next compartment (203).

15. An apparatus according to claim 14, wherein said compartments have cross-sections which are inscribed in the substantially cylindrical casing of the reactor.

16. An apparatus according to one of claims 14 and 15, wherein the reactor casing (10) has a base of circular section.

17. An apparatus according to one of claims 14 to 16, wherein said common partition-wall (210) comprises bearing plane panels.

18. An apparatus according to one of claims 14 to 16, wherein said common partition-wall (210) comprises self-bearing panels of curved shape.

19. An apparatus according to claim 18, wherein the base of the self-bearing panels of curved shape is shaped as an arc of circle of radius ranging from 0.1 to 100 times that of the reactor, or is shaped as a portion of quadratic curve.

20. An apparatus according to any one of claims 14 to 19, wherein said distribution means and said collecting means are arranged substantially parallelly to said generatrix in each compartment.

21. An apparatus according to any one of claims 14 to 20 wherein said inlet (1) and outlet (3) means are respectively located substantially at the level of the reactor first end part (30) and second end part (31), characterized in that the cross-sectional area of the distribution means (20-fig 14), at the level of said first end part, is larger than the cross-sectional area of the distribution means (20-fig 15) at the level of the second end part, and in that the cross-sectional area of the collecting means (21-fig 14) at the level of the first end part is smaller than that of the collecting means (21-fig 15) at the level of the second end part.

22. An apparatus according to any one of claims 14 to 21, wherein said distribution and collecting means comprise at least one distribution and collecting section substantially parallel to said generatrix or at least one distribution and collecting section substantially oblique with respect to said generatrix, each of said sections being staggered with respect to one another in the same direction all along the reactor.

23. An apparatus according to one of claims 14 to 22, wherein said distribution means (20) and said collecting means (21) comprise a plurality of holes (12) arranged along said means and whose respective sections are substantially proportional to the distance between said distribution means and said collecting means.

24. An apparatus according to one of claims 14 to 23, wherein the reactor is horizontal and comprises at least one substantially vertical common partition-wall (210b, fig 1B).

25. An apparatus according to one of claims 14 to 24 comprising cold gas feeding means (4f) provided at the outlet of the reaction effluent of a compartment before the inlet into the next compartment.

26. The use of the process according to one of claims 1 to 13 or of the apparatus according to one of claims 14 to 25 for the ammonia synthesis from hydrogen and nitrogen or for the synthesis of methanol or higher homolog alcohols from hydrogen and carbon oxides or for the catalytic reforming of gasolines.

**Ansprüche**

1. Verfahren zur Ausführung chemischer Reaktionen unter Druck in einer zylindrischen Reaktionszone mit an der Basis kreisförmigem Querschnitt mit wenigstens zwei Kammern, die je wenigstens zum Teil wenigstens einen festen Katalysator aufweisen, wobei man ein Reaktionsfluid in wenigstens eine Kammer einführt, dieses Reaktionsfluid in dieser Kammer zirkulieren läßt, einen Reaktionsabstrom aus dieser Kammer gewinnt, man Wärme zwischen dem Abstrom, der in wenigstens eine nachfolgende Kammer eintreten soll und einem Wärmeaustauschermedium austauscht, man anschließend diesen Abstrom, der Wärme ausgetauscht hat, in diese nachfolgende Kammer einführt, diesen Abstrom in dieser Kammer zirkulieren läßt und einen anderen Reaktionsabstrom aus dieser nachfolgenden Kammer gewinnt, wobei die Reaktionszone von länglicher Form ist und einen Mantel mit wenigstens einer Erzeugenden aufweist, wobei der Mantel einen geschlossenen Querschnitt definiert, der sich in einer diese Erzeugende schneidenden Ebene befindet, wobei das Verfahren dadurch gekennzeichnet ist, daß man das Reaktionsfluid und den Reaktionsabstrom in diese Kammern über wenigstens eine Verteilerzone eintreten läßt, man dieses Reaktionsfluid und diesen Reaktionsabstrom in diesen Kammern quer im wesentlichen senkrecht zu dieser Erzeugenden strömen läßt, wobei diese Kammern von länglicher Gestalt entsprechend dieser Erzeugenden sowie dicht sind, wobei jede dieser Kammern benachbart einer oder zwei anderen Kammern ist und jede Gruppe von zwei benachbarten Kammern eine gemeinsame Wand oder Trennwand entweder im wesentlichen parallel zu dieser Erzeugenden oder im wesentlichen schräg, bezogen auf diese Erzeugende, umfaßt, man den Reaktionsabstrom in wenigstens einer Sammelzone gewinnt, wobei das Verfahren im übrigen dadurch gekennzeichnet ist, daß man Wärme außerhalb der Reaktionszone zwischen dem Abstrom und einem äußeren Wärmeaustauschermedium austauscht.

2. Verfahren nach Anspruch 1, wobei man das Reaktionsfluid im wesentlichen in Höhe eines ersten Endes der Reaktionszone einführt, das Reaktionsfluid und den Reaktionsabstrom in der Verteilerzone allmählich abnehmenden Querschnitts zirkulieren läßt, man diesen Reaktionsabstrom, der die Kammer durchströmt hat, in die Sammelkammer allmählich zunehmenden Querschnitts strömen läßt und den Reaktionsabstrom in Höhe des zweiten Endes der Reaktionszone gewinnt.

3. Verfahren nach einem der Ansprüche 1 und 2, wobei man Wärme austauscht, indem man ein Gemisch dieser Reaktionsabströme mit relativ kaltem Gas herbeiführt.

4. Verfahren nach einem der Ansprüche 1 bis 3, bei dem man dieses Fluid und diesen Abstrom in diesen Kammern derart zirkulieren läßt, daß der Gesamtweg in der Reaktionszone zweimal kleiner als die Länge L dieser Reaktionszone wird.

5. Verfahren nach einem der Ansprüche 1 bis 4, bei dem man das Reaktionslfluid und den Reaktionsabstrom im wesentlichen parallel zu dieser gemeinsamen Hand strömen läßt.

6. Verfahren nach einem der Ansprüche 1 bis 5, wobei man das Reaktionsfluid und den Reaktionsabstrom im wesentlichen senkrecht zu dieser gemeinsamen Hand strömen läßt.

7. Verfahren nach einem der Ansprüche 1 bis 6, bei dem die Reaktionszone horizontal ist.

8. Verfahren nach einem der Ansprüche 1 bis 6, bei dem die Reaktionszone vertikal ist.

9. Verfahren nach Anspruch 7, bei dem man das Reaktionsfluid und den Reaktionsabstrom von oben nach unten in diesen Kammern strömen läßt.

10. Verfahren nach einem der Ansprüche 1 bis 9, bei dem die Reaktionszone wenigstens drei Kammern umfaßt und bei dem man das Reaktionsfluid parallel in zwei Kammern strömen läßt.

11. Verfahren nach einem der Ansprüche 1 bis 9, bei dem man das Reaktionsfluid, dann den Reaktionsabstrom in einer Aufeinanderfolge von Kammern immer größeren Volumens strömen läßt.

12. Verfahren nach einem der Ansprüche 1 bis 9 und 11, bei dem man das Reaktionsfluid und/oder den Reaktionsabstrom in jeder der in Reihe geschalteten Kammern zirkulieren läßt.

13. Verfahren nach einem der Ansprüche 1 bis 12, bei dem die Geschwindigkeit der Gase zwischen 1 m/s und 200 m/s beträgt.

14. Vorrichtung zur Durchführung chemischer Reaktionen unter Druck umfassend :

    – einen Reaktor (2), der in der Lage ist, wenigstens einen Katalysator zu enthalten, der über einen im wesentlichen zylindrischen Mantel (10) verfügt, der durch wenigstens eine Erzeugende definiert ist und ein erstes dichtes Ende (30) und ein zweites dichtes Ende (31) aufweist,

    – wenigstens zwei benachbarten Kammern (201, 203), die in diesem Reaktor länglicher Gestalt entsprechend dieser Erzeugenden enthalten sind, wobei jede Gruppe von zwei benachbarten Kammern eine gemeinsame Wand oder Trennwand (210) umfaßt, jede Kammer im übrigen wenigstens ein Eintrittsmittel (1) für das Reaktionsfluid oder den Reaktionsabstrom in diese Kammer und wenigstens ein Austrittsmittel (3) für den Reaktionsabstrom, der diese Kammer durchströmt hat, umfaßt, wobei diese Eintritts- und Austrittsmittel über diesen Mantel (10) mit der Umgebung außerhalb des Reaktors in Verbindung stehen,

dadurch gekennzeichnet, daß diese Kammern dicht sind, diese trennende oder gemeinsame Wand entweder im wesentlichen parallel zur Erzeugenden oder im wesentlichen schräg hierzu ist und daß der Reaktor aufweist :

    – wenigstens ein Verteilermittel (20) in dieser Kammer, das so ausgebildet ist, daß es quer in dieser Kammer das Reaktionsfluid oder den Reaktionsanstrom im wesentlichen senkrecht zu dieser Erzeugenden verteilt und mit diesem Eintrittsmittel verbunden ist,

    – wenigstens ein Sammlermittel (21) für den Reaktionsabstrom aus jeder Kammer, das mit dem Austrittsmittel für diese Kammer verbunden ist und

    – wenigstens ein äußeres Wärmeübertragungsmittel (4), das zwischen dieses Austrittsmittel (3) für den Reaktionsabstrom aus einer der Kammern und dem Eintrittsmittel (8) des Reaktionsabstroms in diese folgende Kammer (203) zwischengeschaltet ist.

15. Vorrichtung nach Anspruch 14, bei der diese Kammern Schnittquerschnitte aufweisen, die in diesen im wesentlichen zylindrischen Mantel des Reaktors einbeschrieben sind.

16. Vorrichtung nach einem der Ansprüche 14 und 15, bei der der Mantel (10) des Reaktors eine Basis kreisförmigen Querschnitts hat.

17. Vorrichtung nach einem der Ansprüche 14 bis 16, bei der diese Trennwand (210) abgestützte plane Platte umfaßt.

18. Vorrichtung nach einem der Ansprüche 14 bis 16, bei der diese Trennwand (210) Platten selbsttragender Form umfaßt.

19. Vorrichtung nach Anspruch 18, bei der die Platten gekrümmter selbsttragender Form eine Basis entweder als Kreisbogen, dessen Radius zwischen dem 0,1 und 100 fachen desjenigen des Reaktors liegt oder als Teil quadratischer Kurven aufweisen.

20. Vorrichtung nach einem der Ansprüche 14 bis 19, bei der dieses Verteilermittel und dieses Sammlermittel im wesentlichen Parallel zu dieser Erzeugenden in jeder Kammer angeordnet sind.

21. Vorrichtung nach einem der Ansprüche 14 bis 20, bei der diese Eintritts- (1) und Austrittsmittel (3) jeweils im wesentlichen in Höhe des ersten Endes (30) und des zweiten Endes (31) des Reaktors angeordnet sind, dadurch gekennzeichnet, daß der Querschnitt des Verteilermittels (20, Fig. 14) in Höhe dieses ersten Endes größer als der Querschnitt des Verteilermittels (20, Fig. 15) in Höhe des zweiten Endes ist und daß der Querschnitt des Sammlermittels (21, Fig. 14) in Höhe des ersten Endes kleiner als der des Sammlermittels (21, Fig. 15) in Höhe des zweiten Endes ist.

22. Vorrichtung nach einem der Ansprüche 14 bis 21, bei der diese Verteiler und Sammlermittel entweder wenigstens einen Verteiler- und Sammlerquerschnitt im wesentlichen parallel zu dieser Erzeugenden oder wenigstens einen Verteiler- oder Sammlerquerschnitt im wesentlichen schräg zu dieser Erzeugenden umfassen, wobei jeder dieser Querschnitte versetzt bezogen des anderen in der gleichen Richtung längs des ganzen Reaktors ist.

23. Vorrichtung nach einem der Ansprüche 14 bis 22, bei der dieses Verteilermittel (20) und dieses Sammlermittel (21) eine Vielzahl von Öffnungen (12) umfassen, die längs dieser Mittel angeordnet sind und deren jeweilige Querschnitte im wesentlichen proportional zur Entfernung zwischen diesem Verteilermittel und diesem Sammlermittel sind.

24. Vorrichtung nach einem der Ansprüche 14 bis 23, bei der dieser Reaktor horizontal ist und wenigstens eine im wesentlichen vertikale Trennwand (210 b, Fig. 1b) umfaßt.

25. Vorrichtung nach einem der Ansprüche 14 bis 24, mit Einführungsmitteln (4f) für kalte Gase, die am Austritt des Reaktionsabstroms einer Kammer vor seinem Eintritt in die nachfolgende Kammer angeordnet sind.

26. Anwendung des Verfahrens nach einem der Ansprüche 1 bis 13 oder der Vorrichtung nach einem der Ansprüche 14 bis 25 zur Synthese von Ammoniak ausgehend von Wasserstoff und Stickstoff oder zur Synthese des Methanols oder höherer homologer Alkohole ausgehend von Wasserstoff und Kohlenstoffoxiden oder zum katalytischen Reformieren der Benzine.

**FIG.1A**

**FIG.1B**

**FIG.1C**

**FIG.1D**

**FIG.2**

**FIG.4**

EP 0 279 713 B1

FIG.3

FIG.5

FIG.6

FIG.7

FIG.8

FIG.9

FIG.10

16

FIG.11

FIG.12

FIG.13

FIG.14

FIG.15

FIG.16